# EUROPÄISCHE PATENTSCHRIFT

(11) **EP 3 683 564 B1**
(45) Veröffentlichungstag und Bekanntmachung des Hinweises auf die Patenterteilung: **16.03.2022**
(21) Anmeldenummer: 20151088.0
(22) Anmeldetag: 10.01.2020
(51) Int. Cl.: G01N 1/34, G01N 1/40, G01N 21/3563, G01N 1/28, G01N 21/35, G01N 27/64, H01J 49/04, C12Q 1/18, B01D 17/02, B01D 21/00, B01L 3/00

(54) **VERFAHREN ZUR PROBENAUFBEREITUNG AUF EINEM SPEKTROMETRISCHEN PROBENTRÄGER**
METHOD OF SAMPLE PREPARATION ON A SPECTROMETRIC SAMPLE SUPPORT
MÉTHODE DE PRÉPARATION DES ÉCHANTILLONS SUR UN SUPPORT D'ÉCHANTILLON SPECTROMÉTRIQUE

(30) Priorität: 21.01.2019 DE 102019101389
(43) Veröffentlichungstag der Anmeldung: 22.07.2020
(73) Patentinhaber: Bruker Daltonics GmbH & Co. KG, 28359 Bremen (DE)
(72) Erfinder: Voßgröne, Alexander, 28865 Lilienthal (DE)
(74) Vertreter: Boßmeyer, Jens

(56) Entgegenhaltungen:
- EP-A1- 3 376 202
- EP-A2- 1 053 784
- WO-A1-2018/099500

## Beschreibung

### Gebiet der Erfindung

Die Erfindung betrifft ein Verfahren zur Probenaufbereitung auf einem spektrometrischen Probenträger, bei dem (a) ein Probenträger bereitgestellt wird, der eine Anordnung aus vereinzelten Flüssigkeitstropfen umfasst, die jeweils Mikroorganismen-Sedimente umschließen, (b) eine Platte eines saugfähigen Materials bereitgestellt wird, (c) Platte und Probenträger derart zueinander gebracht werden, dass Tropfenflüssigkeit in das saugfähige Material aufgenommen wird, (d) die örtlich mit Flüssigkeit angereicherte Platte vom Probenträger (lotrecht) abgehoben wird, wodurch die flüssigkeitsabgereicherten Mikroorganismen-Sedimente freigelegt werden, und (e) die freigelegten Mikroorganismen-Sedimente für eine spektrometrische Messung präpariert werden.

### Hintergrund der Erfindung

Der Stand der Technik wird im Folgenden mit Bezug auf einen speziellen Aspekt erläutert. Dies soll jedoch nicht als Einschränkung verstanden werden. Nützliche Fortentwicklungen und Änderungen vom aus dem Stand der Technik Bekannten können auch über den vergleichsweise engen Rahmen dieser Einleitung hinaus anwendbar sein und werden sich geübten Praktikern auf diesem Gebiet nach der Lektüre der nachfolgenden Offenbarung umstandslos erschließen.

Frühere Versuche haben gezeigt, dass sich Mikroorganismen, die in einem Tropfen Nährlösung suspendiert sind, auf einer ebenen Fläche schon nach relativ kurzer Stehzeit (oder "Ruhezeit") von bis zu einer Stunde in einer Mikroorganismenablagerung ansammeln. Die dort in einer Art "Biofilm" sedimentierten Mikroorganismen lassen sich behutsam von Restflüssigkeit und verbleibenden suspendierten Teilchen befreien, beispielsweise indem ein saugfähiges Tuch mit den Tropfen in Berührung gebracht wird. Die Art der Mikroorganismen lässt sich nach dieser "Entfeuchtung" mit einer anschließenden massenspektrometrischen Messung zuverlässig nachweisen, siehe internationale Anmeldung WO 2018/099500 A1. Diese Erkenntnis war erstaunlich, da gefunden wurde, dass mit der abgesaugten Flüssigkeit entgegen der Erwartung nicht gleichzeitig das interessierende Mikroorganismen-Zellsediment entfernt wird. Dieser Befund erlaubt die Mikroorganismenzüchtung (oder Bebrütung) zwecks Wachstumsförderung und die Präparation für eine analytische Messung auf ein und demselben Substrat wie zum Beispiel einer Probenträgerplatte für den Einschub in die Ionenquelle eines Massenspektrometers oder einem Objektträger für den Einschub in den Messschacht eines Infrarot-Spektrometers.

Eine ausgereifte wissenschaftliche Erklärung für dieses mikrobielle Verhalten in einem Tropfen auf flachem Untergrund gibt es noch nicht. Es wird aber vermutet, dass physikalische Wechselwirkungen zwischen der Probenträgeroberfläche und den Mikroorganismus-Zellen sowie Adhäsionsprozesse durch die biochemischen und biophysikalischen Eigenschaften der Mikroorganismus-Zelloberfläche für die bevorzugte Anlagerung oder Sedimentierung auf dem Untergrund verantwortlich sind.

In einer Weiterentwicklung der aus WO 2018/099500 A1 bekannten Technik wird in der Anmeldung EP 3 376 202 A1 eine Maske mit Einbuchtungen oder Löchern vorgeschlagen, deren Innenflächen mit den Randbereichen der Tropfen in Berührung gebracht werden und die Tropfenflüssigkeit über Kapillarkräfte seitlich absaugen. Der Mittelpunkt der Tropfen, unter dem sich der größte Teil des Mikroorganismen-Sediments befindet, soll bei diesem Vorgang unberührt bleiben, da befürchtet wurde, dass direkter Kontakt mit dem Sediment zwingend zur Abreicherung der Mikroorganismen ("Stempeleffekt") und damit einhergehend zu Empfindlichkeitseinbußen bei der anschließenden analytischen Messung führen würde.

Das örtlich kontaktlose Abziehen der Flüssigkeit, entweder über die profilierte Maske oder das Aufsetzen einer formstabilen Platte auf einen die Tropfen umgebenden vertikalen Abstandshalter, wie in EP 3 376 202 A1 beschrieben, hat jedoch den Nachteil, dass immer noch eine solche Menge an Restflüssigkeit der Tropfen auf dem Untergrund am Sediment verbleiben kann, dass die anschließende Untersuchung des Mikroorganismen-Sediments dadurch gestört wird und gegebenenfalls weitere Aufreinigungsmaßnahmen erforderlich werden.

Die europäische Anmeldung EP 1 053 784 A2 offenbart das Prozessieren von kleinsten Probenmengen, z.B. in chemischen oder enzymatischen Reaktionen, von Aufreinigungen oder von analytischen Untersuchungen, in kleinsten Flüssigkeitsmengen in der Größenordnung von einem Mikroliter und darunter, insbesondere für sehr große Anzahlen von Proben in simultanen Prozessen. Die Offenbarung beschäftigt sich damit, die bei kleinsten Flüssigkeitsmengen disproportional wachsenden Behälterwandkontaktflächen mit ihren größtenteils schädlichen Einflüssen durch die Verwendung von stehenden (oder hängenden) Tröpfchen als Mikroreaktoren für das Prozessieren zu verringern, wobei die Tröpfchen auf benetzungsfreundlichen (lyophilen) Ankern in ansonsten benetzungsfeindlichen (lyophoben) Oberflächen aufsitzen und daher eine wohldefinierte Wandkontaktfläche besitzen. Die Anker können in gewünschter Weise passiviert oder aber oberflächenaktiv gemacht werden, letzteres, um bestimmte Prozessierungsschritte zu unterstützen. Weitere Ausformungen beinhalten die Integration elektrischer, optischer, fluidischer oder anderer mikrostrukturierter Elemente in den Träger, um das Prozessieren zu unterstützen.

Es besteht daher ein Bedarf, vereinzelte Mikroorganismen-Sedimente hinreichend vollständig von abdeckenden Flüssigkeitstropfen zu trennen und bei diesem Trennvorgang möglichst wenig Material der Mikroorganismen-Sedimente mit der Flüssigkeit zu entfernen. Weitere von der Erfindung zu lösende Aufgaben ergeben sich für den Fachmann ohne weiteres bei der Lektüre der nachfolgenden Offenbarung.

### Zusammenfassung der Erfindung

Die Erfindung betrifft ein Verfahren zur Probenaufbereitung auf einem spektrometrischen Probenträger, aufweisend die Schritte: (i) Bereitstellen des Probenträgers, der eine Anordnung aus vereinzelten Flüssigkeitstropfen umfasst, die jeweils Mikroorganismen-Sedimente umschließen, (ii) Bereitstellen einer Platte eines saugfähigen Materials, (iii) lotrechtes Absenken der Platte auf den Probenträger derart, dass Mikroorganismen-Sedimente und Platte sich berühren, wobei die Flüssigkeitstropfen in das saugfähige Material aufgenommen werden, (iv) Abheben der örtlich mit Tropfenflüssigkeit angereicherten Platte vom Probenträger, wodurch die flüssigkeitsabgereicherten Mikroorganismen-Sedimente freigelegt werden, und, gegebenenfalls nachdem ein etwaig verbliebener Restflüssigkeitsfilm auf den Mikroorganismen-Zellsedimenten eintrocknen konnte, (v) Präparieren der freigelegten Mikroorganismen-Sedimente für eine spektrometrische Messung, beispielsweise eine anschließende Infrarot- oder massenspektrometrische Messung.

In verschiedenen Ausführungsformen kann die Platte ein quellendes saugfähiges Material aufweisen und bis auf einen kurzen Abstand über dem Probenträger abgesenkt werden, so dass ein Überstand der Tropfen mit der Platte in Berührung kommt und von dieser aufgenommen wird, wobei das saugfähige Material örtlich an den Stellen der aufgenommenen Tropfenflüssigkeit aufquillt und dadurch mit den Mikroorganismen-Sedimenten in Berührung kommt. Besonders geeignet für diese Variante hat sich eine Platte aus Löschkarton erwiesen, dessen Material ein entsprechendes Quellverhalten aufweist. Löschkarton ist insbesondere aus der Restaurierung und Archivierung von feuchtigkeitsempfindlichen Kunstwerken bekannt und stellt im Wesentlichen einen neutralen, säurefreien, saugfähigen und fusselarmen Fließkarton mit einem hohen Anteil an Baumwollfasern dar, der mehr als 50%, insbesondere 60%, betragen kann.

Die Wahl des anfänglich noch berührungsfreien Abstands kann sehr einfach dadurch getestet werden, dass ein mit einem abfärbenden Anstrich versehene Platte gegen den Probenträger abgesenkt wird. Sollten sich nach dem Wiederabheben der Platte Farbspuren auf dem Probenträger finden lassen, war der Abstand nicht groß genug, um direktes Aufsetzen zu vermeiden, und muss nachjustiert werden. Abstände von Bruchteilen eines Millimeters, die durch das Quellverhalten eines saugfähigen Materials wie Löschkarton bei örtlicher Flüssigkeitsaufnahme bis zur Berührung mit dem Mikroorganismen-Sediment geschlossen werden können, lassen sich auf diese Weise leicht experimentell einstellen.

In verschiedenen alternativen Ausführungsformen kann die Platte unmittelbar auf den Probenträger aufgesetzt und sachte angedrückt werden, so dass sich Anordnung und Platte vollflächig berühren.

Die Erfindung beruht auf der unerwarteten und damit überraschenden Erkenntnis, dass es möglich ist, eine ebene und fusselarme Platte eines saugfähigen Materials derart gegen einen ebenen Probenträger abzusenken und mit diesem in Berührung zu bringen, ihn teilweise sogar aufzusetzen und leicht anzudrücken, dass Kapillarkräfte den nahezu vollständigen Flüssigkeitsüberstand eines Tropfens in das Material aufnehmen, ohne dass das darunter verborgene Mikroorganismen-Sediment so stark abgereichert wird, dass eine anschließende spektrometrische Untersuchung von dem Probenträger wegen zu geringen Biomaterials keine aussagekräftigen Ergebnisse liefern würde. Obwohl nach dem (lotrechten) Abheben der Platte ein Flüssigkeitsfilm verbleibt, ist dieser so dünn, dass er innerhalb von Sekunden eintrocknet.

Vorzugsweise sind Probenträger und/oder Platte relativ zueinander federgelagert, so dass übermäßige Druckausübung beim Andrücken von der Federung aufgenommen wird. Die elastische Verformung der Federung bewirkt, dass ein gleichmäßiger Anpressdruck der Platte auf dem Probenträger aufrechterhalten wird. Durch geeignete Wahl der Federkonstante kann dieser Druck den Erfordernissen angepasst und so eingestellt werden, dass das Mikroorganismen-Sediment durch die Druckausübung nicht beeinträchtigt wird.

In verschiedenen Ausführungsformen kann eine dem Probenträger zugewandte Plattenfläche vor dem Absenken leicht angefeuchtet werden, beispielsweise mit deionisiertem Wasser oder Methanol/Ethanol, um das Aufnehmen der Tropfenflüssigkeit zu beschleunigen. Die leichte Anfeuchtung und oberflächliche Benetzung der an der äußeren Plattenfläche liegenden Fasern erleichtert insbesondere das Ausbilden einer Flüssigkeitsverbindung oder Flüssigkeitsbrücke zwischen den auf dem Probenträger abgelegten Tropfen und dem Fasergeflecht der Platte, so dass das außenliegende Fasergewebe bereits von Anfang an oberflächenbenetzt ist und nicht erst zeitraubend bei Flüssigkeitskontakt mit den Tropfen auf dem Probenträger benetzt oder durchtränkt werden muss.

Bevorzugt weist die Platte saugfähige Fasern aus Zellstoff und/oder Pflanzenfasern auf, insbesondere Baumwolle, Nitrocellulose, Nylon, Glasfaser, Polyvinylidendifluorid oder Kombinationen daraus. Löschkarton als besonders geeignetes Material kann zum Beispiel eine Mischung aus 60% Baumwollfasern und 40% gebleichtem (pH-neutral hergestelltem und säurefreiem) Zellstoff enthalten.

Übliche Tropfenvolumina können etwa ein bis zwölf Mikrolitern entsprechen. Diese Volumina in gleichförmiger Tropfenform entsprechen etwa einem Durchmesser von zwei bzw. drei Millimetern über der Fläche des Probenträgers.

Insbesondere in der Infrarot-spektrometrischen oder massenspektrometrischen Analyse, zum Beispiel MALDI-Flugzeitanalyse, lassen sich 48, 96, 384 oder 1536 vereinzelte Tropfen in einem vordefinierten (und regelmäßigen) Muster auf dem Probenträger anordnen. Es ist natürlich auch möglich, den Probenträger mit bevorzugten Tropfenflecken auszuzeichnen, zum Beispiel lyophilen Kreisflächen in lyophober Umgebung. Der Durchmesser der Tropfen wird sich dann entsprechend der Abmessung der lyophilen Kreisfläche einstellen wie bei standardisierten Ankerplatten des AnchorChip^{™}-Typs.

Die Tropfen können insbesondere hauptsächlich Flüssigkeit enthalten, die in Probenvorbereitungsverfahren und Probenaufbereitungsverfahren für die Infrarot-Spektrometrie oder Massenspektrometrie zum Einsatz kommt. Die Tropfen können zum Beispiel Nährlösung aufweisen, und das sedimentierte Material kann in diesen Nährlösungstropfen gezüchtete und dann abgesetzte Mikroorganismen umfassen. Eine Trennung der Nährlösung von sedimentierten Mikroorganismen kann insbesondere als Aufbereitungsschritt einer Probe für eine Infrarot-spektrometrische oder massenspektrometrische Identifizierung (z.B. mittels IR-Transmissionsspektroskopie oder MALDI-Flugzeit-Massenspektrometrie) nach Art/Unterart oder eine anderweitige Charakterisierung der Mikroorganismen wie zum Beispiel der schnellen Bestimmung von Resistenz/Empfindlichkeit der Mikroorganismen gegenüber antimikrobiellen Substanzen wie Antibiotika und Antimykotika dienen.

Gegebenenfalls kann der Nährlösung eine antimikrobielle Substanz beigemischt sein, beispielsweise um durch Bebrütung in dem antibiotika- oder antimykotika-angereicherten Nährlösungstropfen die minimale Hemmkonzentration von Mikroorganismen zu ermitteln.

Zusätzlich oder alternativ können die Tropfen für ein Probenaufbereitungsverfahren zu verwendende Waschflüssigkeit (etwa eine wässerige Lösung oder reines deionisiertes Wasser) oder andere flüssige Arbeitsmedien enthalten. Beispielsweise kann die Waschflüssigkeit von Probenflecken, die mit vorher getrocknetem Mikroorganismen-Sediment oder mit getrockneter Matrixsubstanz und Mikroorganismen-Sediment belegt sind, mit der Platte aufgenommen werden. Vorzugsweise wird die Waschflüssigkeit so gewählt, dass die Matrixsubstanz bzw. das Matrixkristallgitter mit eingebauten Probenkristallen nicht aufgelöst wird, z.B. bei einer α-Cyano-4-Hydroxyzimtsäuren-Affinitätspräparation zur in-situ Entsalzung (siehe Gobom et al., Anal. Chem. 73, 2001, 434-438).

In verschiedenen Ausführungsformen kann eine Metall-, Glas- oder Keramikplatte als spektrometrischer Probenträger verwendet werden. In Betracht kommen insbesondere polierte Edelstahlplatten oder Abwandlungen davon, wie die sogenannten Ankerplatten (AnchorChip^{™}; Bruker Daltonik GmbH), die auf einem Edelstahlsubstrat abwechselnd gegeneinander abgegrenzte lyophile und lyophobe Flächen enthalten. Möglich ist auch eine Glasplatte als Infrarot-spektrometrischer Objektträger für eine Transmissionsmessung. Der Probenträger kann wiederverwendbar oder ein Einwegartikel sein.

Ein üblicher, standardisierter Probenträger für Ionisierung mittels matrix-unterstützter Laserdesorption (MALDI) kann eine regelmäßige Anordnung von 48, 96, 384 oder 1536 Probenflecken umfassen. Die Abmessungen eines Probenträgers und einer Platte saugfähigen Materials können je 127,76 mm (Länge) × 85,48 mm (Breite) entsprechend einer Mikrotiterplatte bei einer Dicke von etwa zwei bis fünf Millimetern betragen, sofern sie deckungsgleich sind. Die Probenfleckenanordnung und damit die Anordnung der Tropfen auf dem Probenträger nimmt jedoch eine geringere Fläche ein als der Probenträger selbst.

Es kann ein Rahmen für die Platte oder ein Schacht für den Probenträger, in den die Platte eingeführt wird, vorgesehen werden, der bei der Führung und Ausrichtung der Platte während des Absenkens auf die Tropfenanordnung behilflich ist, wie es in EP 3 376 202 A1 beschrieben ist, deren Inhalt hier durch Bezugnahme vollinhaltlich eingeschlossen sein soll. Der Rahmen kann beispielsweise fest mit der Platte verbunden werden, insbesondere indem ein überstehender zugeschnittener Rand des saugfähigen Materials gefalzt und dann mit einem Kunststoff getränkt wird, der dann aushärtet. Der Rahmen kann auch an den äußeren Umfang der Platte unter Verwendung eines Spritzkunststoffs angespritzt werden.

Vorzugsweise ist die Platte aus einem steifen und fusselarmen Gewebe gefertigt. Pflanzenfasern und/oder Zellstoff bieten sich dafür an. Sie kann beispielsweise Fasermaterialien wie Baumwollfaser, Nitrocellulose, Nylon, Glasfaser, Polyvinylidendifluorid oder Kombinationen daraus aufweisen, beispielsweise Löschkarton mit etwa 60% Baumwollfasern und 40% gebleichtem (pH-neutral hergestelltem und säurefreiem) Zellstoff. Formbeständige Materialien eignen sich insbesondere für automatisierte Verfahren zum Aufnehmen von Flüssigkeit. Die formbeständigen Platten lassen sich leicht von speziell angepassten Handhabungsvorrichtungen wie zum Beispiel Roboterarmen einem Vorratslager entnehmen, in eine angepasste Halterung einspannen, dem Flüssigkeitsaufnahmeschritt zuführen und dann an einem Ablageort ablegen, ggfs. auch entsorgen.

Bezüglich der Herstellung der Platte ist es möglich, eine Bahn des saugfähigen Materials bereitzustellen und dann Plattenstücke gemäß der gewünschten Außenkontur entsprechend abzulängen. Für die Benutzung in einem mikrobiologischen Labor ist es zweckmäßig, die äußeren Abmessungen der Platte so zu wählen, dass die aufgenommene Flüssigkeit mit eventuell darin verbleibenden, suspendierten Mikroorganismen nicht bis zur Plattenschmalseite oder zur Plattenoberseite vordringen kann, damit die örtlich flüssigkeitsangereicherte Platte ohne Kontaminationsgefahr dort gegriffen und bewegt werden kann.

### Kurze Beschreibung der Abbildungen

Zum besseren Verständnis der Erfindung wird auf die folgenden Abbildungen verwiesen. Die Elemente in den Abbildungen sind nicht unbedingt maßstabsgetreu dargestellt, sondern sollen in erster Linie die Prinzipien der Erfindung (größtenteils schematisch) veranschaulichen. In den Abbildungen sind einander entsprechende Elemente in den verschiedenen Ansichten durch gleiche Bezugszeichen gekennzeichnet.

Abbildungen 1A bis 1E zeigen schematisch ein Ausführungsbeispiel für ein Verfahren, bei dem eine Platte saugfähigen Materials auf einen Tropfen-tragenden Probenträger aufgesetzt wird.

Abbildungen 2A bis 2D veranschaulichen schematisch eine Abwandlung des Verfahrens, bei dem eine Federung überschüssige Druckausübung beim Andrücken der Platte auf den Probenträger aufnimmt und somit für eine gleichmäßige Druckbeaufschlagung sorgt.

Abbildung 3 zeigt schematisch ein Ausführungsbeispiel für ein Verfahren, bei dem eine Platte aus quellendem Material bis auf einen kleinen Abstand auf den Probenträger abgesenkt wird.

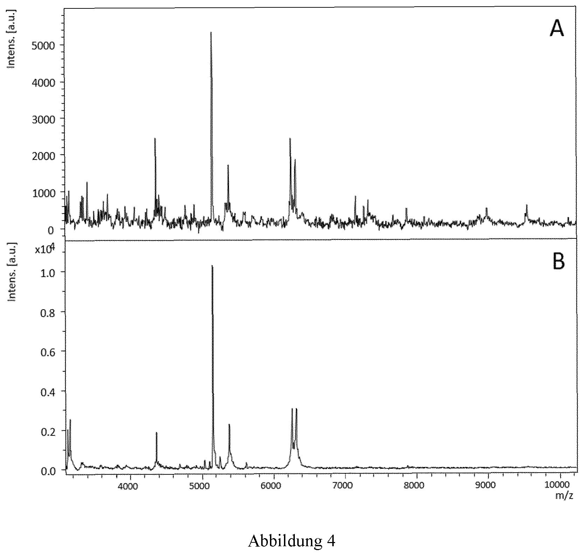

Abbildung 4 zeigt Spektren der MALDI-Flugzeit-Massenspektrometrie nach der Entfernung von Kulturüberständen von einem Enterobacteriales-Sediment, einmal unter Verwendung eines berührungsfreien Verfahrens, oberes Diagramm A, wie es beispielsweise aus E. A. Idelevich et al., Clin. Microbiol. Infect. 2018 (7): 738-743 bekannt ist, wo Whatman^{®} Cellulose Filterpapier als saugfähiges Material eingesetzt wurde, und einmal unter Verwendung eines kontaktierenden Verfahrens gemäß Prinzipien der Erfindung, unteres Diagramm B; vertikale Achse: Intensität in willkürlichen Einheiten; horizontale Achse: ladungsbezogene Masse m/z.

### Detaillierte Beschreibung

Während die Erfindung anhand einer Anzahl von Ausführungsformen dargestellt und erläutert wurde, werden Fachleute auf dem Gebiet anerkennen, dass verschiedene Änderungen in Form und Detail daran vorgenommen werden können, ohne vom Umfang der in den beigefügten Patentansprüchen definierten technischen Lehre abzuweichen.

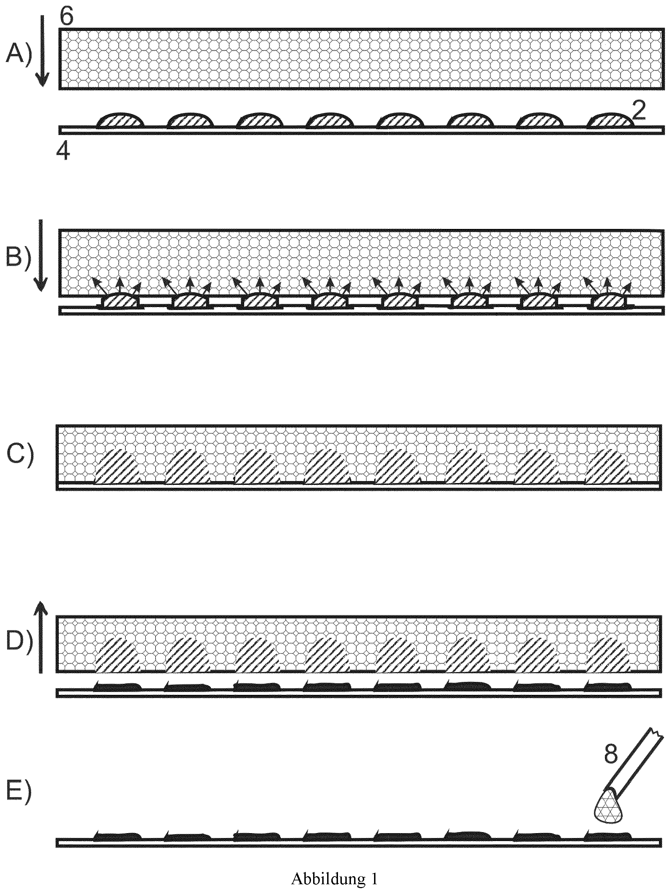

Abbildung 1A zeigt eine Anordnung von Tropfen (2) entlang einer Achterreihe auf einem Probenträger (4), der einer MALDI-Probenträgerplatte oder einem für die Infrarot-Spektrometrie geeigneten Objektträger entsprechen kann. Über dem Probenträger (4) ist eine Platte (6) eines saugfähigen Materials in Stellung gebracht. Die Platte (6) wird langsam lotrecht gegen den Probenträger (4) bewegt, wobei die Tropfen (2) mit dem saugfähigen Material der Platte (6) ab einem bestimmten Absenkpunkt in Berührung kommen, so dass die Tropfenflüssigkeit von dem Material der Platte (6) über Kapillarkräfte aufgenommen wird, Abbildung 1B. Im Unterschied zu früheren Offenbarungen kann diese Absenkbewegung mit einem Aufsetzen der Platte (6) auf den Probenträger (4) enden wie dargestellt, Abbildung 1C. Auf diese Weise kann eine vollflächige Berührung zwischen Platte (6) und Tropfenanordnung (2) hergestellt werden, die weniger Restflüssigkeit von den Tropfen (2) auf den Mikroorganismen-Sedimenten hinterlässt und damit folgende spektrometrische Messungen der Sedimente erleichtert.

Überraschenderweise hat sich herausgestellt, dass ein zuvor vermuteter Stempeleffekt, gemäß dem viele Mikroorganismen-Zellen bei unmittelbarer Berührung mit dem saugfähigen Material der Platte (6) durch Übertragung auf diese aus dem Sediment entfernt werden und dadurch eine anschließende Messung wegen der Verringerung des verfügbaren Biomaterials erschweren, weshalb in früheren Offenbarungen ein kontaktloses Abziehen der Flüssigkeit befürwortet wurde, deutlich geringer ausgeprägt ist als befürchtet. Zwar haben Experimente im Hause der Anmelderin, in denen die zum Flüssigkeitsaufnehmen verwendeten Platten bebrütet wurden, ergeben, dass Mikroorganismenkolonien auf der Plattenfläche an den Stellen entstehen können, wo Tropfenflüssigkeit aufgenommen wurde, jedoch erwies sich die Menge des "abgestempelten" Biomaterials entgegen der Erwartung als so gering, dass die folgenden spektrometrischen Untersuchungen des auf dem Probenträger (4) verbliebenden Materials nicht beeinträchtigt wurden.

Eine ausgereifte wissenschaftliche Erklärung für diese vorteilhaften Eigenschaften konnte noch nicht gefunden werden. Es wird jedoch vermutet, dass die Wechselwirkung zwischen Fasern des Plattenmaterials und Mikroorganismen sehr gering ist, so dass beide nicht gut aneinander haften, was dem Stempeleffekt entgegenwirkt. Ferner wird angenommen, dass das Einschränken der Plattenbewegung auf ein rein lotrechtes Absenken auf den Probenträger unter Vermeidung von seitlichen Scherbewegungen, im Gegensatz zu der früheren Offenbarung, wo seitliche Bewegungen durchaus empfohlen wurden, auch die Gefahr einer Verschmierung des Mikroorganismen-Biofilms, die zur Umlagerung von Zellen vom Probenträger an das Plattenmaterial führen könnte, verringert und gegebenenfalls beseitigt.

Nach Berührung erfolgt die Aufnahme der Tropfenflüssigkeit, gegebenenfalls begünstigt durch vorherige leichte Anfeuchtung des Plattenmaterials, in sehr kurzer Zeit; üblicherweise Bruchteilen einer Sekunde. Danach kann die örtlich flüssigkeitsangereicherte Platte (6) wieder angehoben und entfernt werden, Abbildung 1D. Die aufgenommene Flüssigkeit wird sicher in dem Kapillargeflecht der Platte (6) gehalten, so dass keine Gefahr besteht, dass sie beim (lotrechten) Abheben wieder heraustropft und den Probenträger (4) verunreinigt. Vielmehr handelt es sich um eine sehr sichere und verlässliche Art der Flüssigkeitsentfernung. Die teils vollgesaugte Platte (6) wird als Verbrauchsmaterial typischerweise entsorgt, was bei mikrobiologischen Anwendungen von Vorteil ist. Sie könnte gegebenenfalls aber auch waschbar und dann wiederverwendbar sein.

Wie bereits zuvor ausgeführt, werden die von den Tropfen (2) umschlossenen Mikroorganismen-Sedimente durch das sanfte Aufnehmen der Flüssigkeit mittels Kapillarkräften nicht entfernt, sondern verbleiben größtenteils mittig auf der Oberfläche des Probenträgers (4), wo die Tropfen (2) abgelegt waren. Das sedimentierte und nun von Flüssigkeit befreite Material steht damit weiterer Bearbeitung zur Verfügung wie beispielsweise einer Probenpräparation für die Infrarot-Spektrometrie, die Ionisierung mittels matrix-unterstützter Laserdesorption oder ähnlichen Prozessierschritten, angedeutet durch Aufpipettieren einer Arbeitsflüssigkeit, zum Beispiel Matrixsubstanz für MALDI, bei (8) in Abbildung 1E.

Der Abstand vom Plattenrand zum nächstgelegenen Tropfen entspricht vorzugsweise mindestens dem Tropfenradius auf der Fläche des Probenträgers, sicherer ist der Tropfendurchmesser, um zu vermeiden, dass bei der Flüssigkeitsaufnahme in das saugfähige Material biogefährliche Bestandteile zu nahe an den Rand gesogen werden, wo die Platte vom Laborpersonal gegebenenfalls gegriffen wird. Ein ähnlicher Sicherheitsabstand sollte für die Mindestdicke der Platte eingehalten werden, um einem Durchwirken der aufgenommenen Flüssigkeit zu der Plattenfläche, die von der Flüssigkeitsaufnahme abgewandt ist, vorzubeugen.

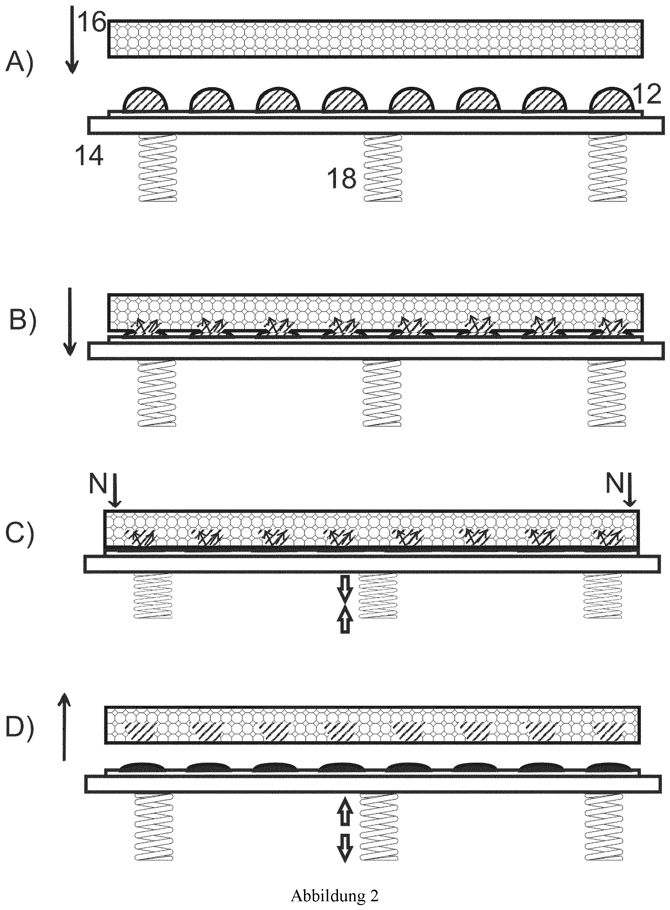

In einer Variante des zuvor erläuterten Verfahrens kann die Platte (16) nach dem Absenken gegen den und Aufsetzen auf dem Probenträger (14) auch leicht angedrückt werden, um eine vollflächige Berührung von Platte (16) und Anordnung (12) oder Probenträgeroberfläche, die die vollständigste Flüssigkeitsaufnahme erlaubt, zu gewährleisten. Um insbesondere bei händischem Andrücken zu vermeiden, dass ein übermäßiger Druck ausgeübt wird (beispielsweise von unerfahrenem Laborpersonal), der das Mikroorganismen-Sediment beeinträchtigen kann, können Platte (16) und Probenträger (14) relativ zueinander federgelagert sein, wie durch die Wendelfedern (18) in den Abbildungen 2A bis 2D schematisch angedeutet. Es versteht sich, dass der Probenträger (14), die Platte (16) oder auch beide Elemente federgelagert sein können, um den gewünschten Effekt zu erzielen, wenngleich in der Sequenz aus Abbildung 2 nur die Federung des Probenträgers (14) illustriert ist.

Eine Platte (16) eines saugfähigen Materials wird gegen den die Flüssigkeitstropfen (12) tragenden Probenträger (14) abgesenkt, Abbildung 2A. In diesem Zustand ist die Federung (18) entspannt. Sobald die Platte (16) mit dem Tropfenüberstand in Berührung kommt, was noch vor der Berührung mit der Probenträgeroberfläche geschieht, wird die Tropfenflüssigkeit in das saugfähige Material der Platte (16) aufgenommen, Abbildung 2B. Auch in diesem Zustand ist die Federung (18) noch entspannt. Sobald die Platte (16) auf dem Probenträger (14) aufsetzt, wird Widerstand bei der Absenkbewegung spürbar. Nun kann Druck ausgeübt werden, um eine gleichmäßige und insbesondere vollflächige Berührung von Platte (16) und Probenträgeroberfläche oder Anordnung (12) zu gewährleisten und Restflüssigkeit so weit wie möglich aufzunehmen, Abbildung 2C. Die Federung (18) ist jetzt gemäß des ausgeübten Drucks N und der Federkonstanten zusammengedrückt und verhindert somit exzessiven Druck auf die Mikroorganismen-Sedimente. Nach einer Zeitspanne von Bruchteilen einer Sekunde, wenn die Flüssigkeit in das Plattenmaterial aufgenommen ist, kann die Platte (16) wieder lotrecht abgehoben werden und legt somit die flüssigkeitsabgereicherten Mikroorganismen-Sedimente frei, die dann weitergehender Bearbeitung auf dem Probenträger (14) zugänglich sind, Abbildung 2D. Die Federung (18) entspannt sich dabei wieder.

Es versteht sich, dass die Wendelfedern (18) aus dem vorgenannten Beispiel lediglich der Illustrierung des Federungsprinzips dienen. Es können in den hier vorgestellten Verfahren auch andere Federungsmechanismen zu Einsatz kommen, wie es ein Praktiker für zweckmäßig hält.

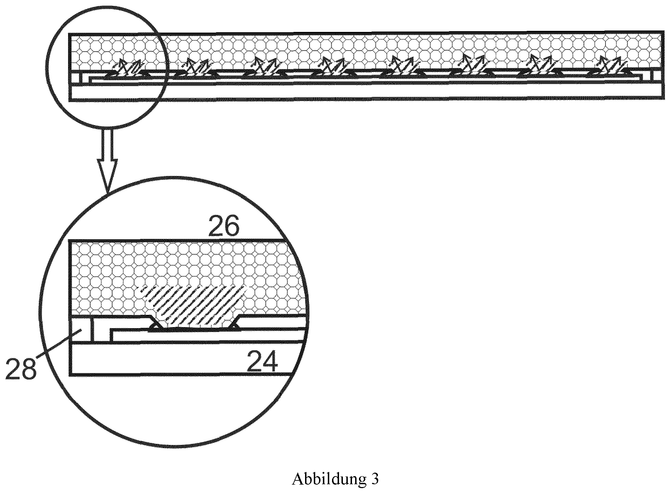

Vorstehend wurden Ausführungsformen beschrieben, in denen die saugfähige Platte (26) unmittelbar auf dem Probenträger (24) aufgesetzt wird. Es ist jedoch auch möglich, die Platte (26) leicht über dem Probenträger (24) auf Abstand zu halten, zum Beispiel durch Absetzen auf einem seitlichen Grat (28), wenn sie aus einem quellenden saugfähigen Material gefertigt ist, Abbildung 3. Durch die Berührung der Plattenfläche mit den Tropfenüberständen wird Flüssigkeit örtlich in die Platte (26) aufgenommen und führt dort zu einer Volumenvergrößerung ("Aufquellung"), so dass das aufgequollene Material mit den Mikroorganismen-Sedimenten sachte in Berührung kommt, siehe den vergrößerten Ausschnitt in Abbildung 3. Es hat sich herausgestellt, dass durch diese sachte Berührung das Sediment nicht weiter beeinträchtigt wird, insbesondere werden nur wenige Mikroorganismen-Zellen auf die Plattenfläche übertragen ("Stempeleffekt"), jedoch erlaubt sie es, die Tropfenflüssigkeit vollständiger aufzunehmen, als es mit den früher veröffentlichten, durch Verwendung nicht-quellender Materialien streng auf Abstand basierenden Techniken möglich ist. Die Menge an Restflüssigkeit, die nach dem (lotrechten) Abheben der örtlich vollgesaugten Platte (26) auf dem Probenträger (24) verbleibt wird dadurch verringert, was die nachfolgenden spektrometrischen Messungen erleichtert.

Um die Handhabung der Platte zu vereinfachen, kann sie in einen Rahmen eingelegt oder eingespannt sein. Der Rahmen lässt sich beispielsweise so dimensionieren, dass er einen Probenträger, auf dem sich ein Tropfenfeld befindet, bündig umschließt, wie in EP 3 376 202 A1 beschrieben. Er lässt sich als Einwegartikel auslegen, der zusammen mit der vollgesaugten Platte entsorgt wird, oder auch waschbar und wiederverwendbar sein. Mögliche Ausgestaltungen umfassen einen Rahmen mit abgestufter Innenkontur, auf der die Platte reibschlüssig ablegbar ist. Gleitet der Rahmen um die äußere Probenträgerkontur herunter, wird ab einem bestimmten Punkt der Flüssigkeitskontakt hergestellt. Der Rahmen hat ferner den Vorteil, für eine verlässliche Ausrichtung und Führung der Platte relativ zum Tropfenfeld zu sorgen.

In einer alternativen Ausführungsform, ebenfalls vom Prinzip her in EP 3 376 202 A1 beschrieben, kann der Rahmen fest mit der Platte verbunden werden. Beispielsweise lässt sich ein überstehender zugeschnittener Rand des saugfähigen Materials falzen und dann mit einem Kunststoff tränken, welcher dann aushärtet, um Festigkeit und Formstabilität zu gewährleisten (einstückige Variante). Der Rahmen kann gegebenenfalls auch am äußeren Umfang an die Platte unter Verwendung eines Spritzkunststoffs angespritzt werden.

Ebenfalls aus EP 3 376 202 A1 bekannt ist, einen Probenträger, der das Tropfenfeld trägt, in einen allseitig umschließenden Schacht einzulegen. Die Platte kann dann ähnlich dimensioniert sein wie der Probenträger und von der oberen Schachtöffnung an abwärts langsam auf den Probenträger heruntergleiten. Griffmulden an den Schachtwänden können das Einlegen und Herausheben von Probenträger und Platte erleichtern.

Abbildung 4 zeigt Messdaten, die die Vorteile des hier beschriebenen Verfahrens verdeutlichen. Die (nahezu) vollständige Entfernung eines Tropfenüberstands, hier Kultur- oder Nährlösungsüberstand nach Bakterienbebrütung unmittelbar auf einem MALDI-Probenträger, gemäß Prinzipien der vorliegenden Offenbarung, unteres Diagramm B, und damit auch die Entfernung von Kontaminationen, z.B. Bestandteilen des Nährmediums oder Salzen, die die anschließende Messung stören können, führt zu einer verbesserten Qualität der Messdaten in Form von höheren Intensitäten der Ionensignale sowie einem verbesserten Signal-zu-Rausch-Verhältnis.

Bezüglich einer Bestimmung der antibiotischen Suszeptibilität (Cephalosporin-Antibiotikum: Ceftazidim) von untersuchten Gram-negativen Bakterien lieferten das erfindungsgemäße kontaktierende Verfahren und das berührungsfreie Verfahren aus dem Stand der Technik nach 4h / 4,5h / 5h Bebrütung übereinstimmende Ergebnisse im Vergleich zu einem üblichen Nährlösungs-Mikroverdünnungsverfahren (20h Bebrütung) zu 95% / 100% / 100% bzw. 93% / 93% / 92%, was den hohen Nutzen dieser Abläufe unterstreicht.

Neben den beispielhaft erläuterten Ausführungen sind noch weitere Ausführungsformen der Erfindung denkbar. In Kenntnis dieser Offenbarung ist es dem Fachmann ohne weiteres möglich, weitere vorteilhafte Aufbereitungsverfahren von Proben für eine Infrarotspektrometrische oder massenspektrometrische Messung unter Verwendung eines desorbierenden Ionisierungsverfahrens zu entwerfen, die vom Schutzbereich der Patentansprüche unter Einschluss etwaiger Äquivalente umfasst sein sollen.

## Patentansprüche

1. Verfahren zur Probenaufbereitung auf einem spektrometrischen Probenträger, aufweisend die Schritte:
- Bereitstellen des Probenträgers, der eine Anordnung aus vereinzelten Flüssigkeitstropfen umfasst, die jeweils Mikroorganismen-Sedimente umschließen,
- Bereitstellen einer Platte eines saugfähigen Materials,
- lotrechtes Absenken der Platte auf den Probenträger derart, dass Mikroorganismen-Sedimente und Platte sich berühren, wobei die Flüssigkeitstropfen in das saugfähige Material aufgenommen werden,
- Abheben der örtlich mit Tropfenflüssigkeit angereicherten Platte vom Probenträger, wodurch die flüssigkeitsabgereicherten Mikroorganismen-Sedimente freigelegt werden, und
- Präparieren der freigelegten Mikroorganismen-Sedimente für eine spektrometrische Messung.

2. Verfahren nach Anspruch 1, bei dem die Platte ein quellendes saugfähiges Material aufweist und bis auf einen kurzen Abstand über dem Probenträger abgesenkt wird, so dass ein Überstand der Tropfen mit der Platte in Berührung kommt und von dieser aufgenommen wird, wobei das saugfähige Material örtlich an den Stellen der aufgenommenen Tropfenflüssigkeit aufquillt und mit den Mikroorganismen-Sedimenten in Berührung kommt.

3. Verfahren nach Anspruch 1, bei dem die Platte unmittelbar auf den Probenträger aufgesetzt und sachte angedrückt wird, so dass sich Anordnung und Platte vollflächig berühren.

4. Verfahren nach Anspruch 3, bei dem Probenträger und/oder Platte relativ zueinander federgelagert sind, so dass übermäßige Druckausübung beim Andrücken von der Federung aufgenommen wird.

5. Verfahren nach einem der Ansprüche 1 bis 4, bei dem eine dem Probenträger zugewandte Plattenfläche vor dem Absenken leicht angefeuchtet wird, um das Aufnehmen der Tropfenflüssigkeit zu beschleunigen.

6. Verfahren nach einem der Ansprüche 1 bis 5, bei dem die Platte saugfähige Fasern aus Zellstoff und/oder Pflanzenfasern aufweist.

7. Verfahren nach einem der Ansprüche 1 bis 6, bei dem ein Tropfenvolumen etwa ein bis zwölf Mikrolitern entspricht.

8. Verfahren nach einem der Ansprüche 1 bis 7, bei dem die Mikroorganismen-Sedimente in Flüssigkeitstropfen aus Nährlösung oder Waschflüssigkeit bereitgestellt werden.

9. Verfahren nach Anspruch 8, bei dem der Nährlösung eine antimikrobielle Substanz beigemischt ist.

10. Verfahren nach einem der Ansprüche 1 bis 9, bei dem sich dem Präparieren der freigelegten Mikroorganismen-Sedimente eine infrarot- oder massenspektrometrische Messung von dem Probenträger anschließt.

11. Verfahren nach einem der Ansprüche 1 bis 10, bei dem zwischen Abheben der Platte und Präparieren eine kurze Zeit gewährt wird, um einen etwaig verbliebenen Restflüssigkeitsfilm auf den Mikroorganismen-Zellsedimenten eintrocknen zu lassen.

## Claims

1. A method for sample preparation on a spectrometric sample support, comprising the steps:
- Providing the sample support, which contains an arrangement of individual droplets of liquid, each of which has microorganism sediments enclosed in it,
- Providing a plate of absorbent material,
- Lowering the plate vertically onto the sample support in such a way that the microorganism sediments and plate come into contact, wherein the droplets of liquid are absorbed into the absorbent material,
- Lifting the plate, which is locally enriched with droplet liquid, off the sample support, whereby the microorganism sediments that are depleted of liquid are exposed, and
- Preparing the exposed microorganism sediments for spectrometric measurement.

2. The method according to Claim 1, wherein the plate contains a swelling absorbent material and is lowered until there is only a small gap between it and the sample support so that a supernatant of the droplets comes into contact with the plate and is absorbed by it, wherein the absorbent material undergoes localized swelling at the sites where the droplet liquid is absorbed and makes contact with the microorganism sediments.

3. The method according to Claim 1, wherein the plate is placed directly onto the sample support and gently pressed against it so that the arrangement and the plate make all-over contact.

4. The method according to Claim 3, wherein the sample support and/or the plate are spring-mounted with relative respect to each other so that any excessive pressure applied, when the plate is pressed down, is taken up by the springs.

5. The method according to any one of the Claims 1 to 4, wherein a plate surface facing toward the sample support is moistened slightly before being lowered to accelerate the absorption of liquid from the droplets.

6. The method according to any one of the Claims 1 to 5, wherein the plate has absorbent fibers of cellulose and/or plant fibers.

7. The method according to any one of the Claims 1 to 6, wherein the volume of a droplet corresponds approximately to one to twelve microliters.

8. The method according to any one of the Claims 1 to 7, wherein the microorganism sediments are provided in liquid droplets of nutrient solution or washing liquid.

9. The method according to Claim 8, wherein an antimicrobial substance is admixed to the nutrient solution.

10. The method according to any one of the Claims 1 to 9, wherein the preparation of the exposed microorganism sediments is followed by an infrared or mass spectrometric measurement from the sample support.

11. The method according to any one of the Claims 1 to 10, wherein there is a short wait between lifting off the plate and the preparation to allow any remaining residual liquid film on the microorganism cell sediments to dry.

## Revendications

1. Procédé de préparation d'échantillons sur un support d'échantillons spectrométrique, comprenant les étapes suivantes :
- Fourniture du support d'échantillon comprenant un ensemble de gouttes de liquide individualisées, chacune renfermant des sédiments de microorganismes,
- Mise à disposition d'une plaque de matériau absorbant,
- Abaissement vertical de la plaque sur le support d'échantillon de manière à ce que les sédiments de microorganismes et la plaque se touchent, les gouttes de liquide étant absorbées dans le matériau absorbant,
- Soulèvement de la plaque localement enrichie de gouttes de liquide du support d'échantillon, ce qui libère les sédiments de microorganismes appauvris en liquide, et
- Préparation des sédiments de microorganismes libérés pour une mesure spectrométrique.

2. Procédé selon la revendication 1, dans lequel la plaque comprend un matériau absorbant gonflant et est abaissée à une courte distance au-dessus du support d'échantillon, de sorte qu'un surnageant des gouttes entre en contact avec la plaque et est absorbé par celle-ci, le matériau absorbant gonflant localement aux endroits où le liquide des gouttes est absorbé et entre en contact avec les sédiments de microorganismes.

3. Procédé selon la revendication 1, dans lequel la plaque est placée directement sur le support d'échantillon et est pressée doucement, de sorte que l'ensemble et la plaque entrent en contact sur toute leur surface.

4. Procédé selon la revendication 3, dans lequel le support d'échantillon et/ou la plaque sont montés sur ressort l'un par rapport à l'autre, de sorte que la pression potentiellement excessive exercée lors de l'application est absorbée par le ressort.

5. Procédé selon l'une quelconque des revendications 1 à 4, dans lequel une surface de la plaque tournée vers le support d'échantillon est légèrement humidifiée avant l'abaissement afin d'accélérer l'absorption du liquide de goutte.

6. Procédé selon l'une quelconque des revendications 1 à 5, dans lequel la plaque comprend des fibres absorbantes de cellulose et/ou de fibres végétales.

7. Procédé selon l'une quelconque des revendications 1 à 6, dans lequel un volume de goutte correspond à environ un à douze microlitres.

8. Procédé selon l'une quelconque des revendications 1 à 7, dans lequel les sédiments de microorganismes sont fournis dans des gouttes liquides de solution nutritive ou de liquide de lavage.

9. Procédé selon la revendication 8, dans lequel une substance antimicrobienne est mélangée à la solution nutritive.

10. Procédé selon l'une quelconque des revendications 1 à 9, dans lequel la préparation des sédiments de microorganismes libérés est suivie d'une mesure du support d'échantillon par spectrométrie infrarouge ou spectrométrie de masse.

11. Procédé selon l'une quelconque des revendications 1 à 10, dans lequel on laisse un court laps de temps entre le soulèvement de la plaque et la préparation pour laisser sécher un éventuel film de liquide résiduel sur les sédiments cellulaires de microorganismes.
